# EUROPÄISCHE PATENTSCHRIFT

(11) **EP 3 941 220 B1**
(45) Veröffentlichungstag und Bekanntmachung des Hinweises auf die Patenterteilung: **18.01.2023**
(21) Anmeldenummer: 20739833.0
(22) Anmeldetag: 10.07.2020
(51) Int. Cl.: A23L 33/10, A23L 33/105, A23L 33/12, A23L 33/125

(54) **NAHRUNGSERGÄNZUNGSMITTEL UND NAHRUNGSMITTEL**
FOOD SUPPLEMENT AND FOOD
COMPLÉMENT ALIMENTAIRE ET ALIMENT

(30) Priorität: 09.06.2020 DE 102020115238
(43) Veröffentlichungstag der Anmeldung: 26.01.2022
(73) Patentinhaber: PM-International AG, 5445 Schengen (LU)
(72) Erfinder: SORG, Rolf, 5444 Schengen (LU); MESSER, Wilhelm, 67098 Bad Dürkheim (DE); LAUINGER, Christian, 76275 Ettlingen (DE)
(74) Vertreter: Zech, Stefan Markus
(86) Internationale Anmeldenummer: PCT/EP2020/069513
(87) Internationale Veröffentlichungsnummer: WO 2021/249658

(56) Entgegenhaltungen:
- WO-A1-2016/044813
- DE-A1-102008 056 312
- DE-U1-202011 105 533
- US-A1- 2010 303 961

## Beschreibung

DE202011105533 U1 offenbart eine Vitalstoff-Zusammensetzung gemäß asiatischer und mediterraner Ernährungskonzepte, umfassend Vitamine, Spurenelemente, und Omega-3-Fettsäuren, dadurch gekennzeichnet, dass die Zusammensetzung in wenigstens zwei getrennt vorliegenden Darreichungsformen unterschiedlicher Zusammensetzung vorliegt, wobei eine erste Darreichungsform als weitere wirksame Bestandteile wenigstens einen Extrakt ausgewählt aus der Gruppe umfassend Weinreben-, Oliven-, und/oder Tomaten-Extrakt umfasst und wobei eine zweite Darreichungsform als weitere wirksame Bestandteile wenigstens einen Extrakt ausgewählt aus der Gruppe umfassend Gojibeeren-, Ingwer- und/oder Grüntee-Extrakt umfasst.

Die Erfindung betrifft Nahrungsergänzungsmittel und Nahrungsmittel. Wie Nahrungsmittel werden auch Nahrungsergänzungsmittel üblicherweise oral appliziert. Zur Verbesserung der Aufnahme der Inhaltsstoffe im Gastrointestinatrakt, zur Vereinfachung der Einnahme des Nahrungsergänzungsmittels und zur Vermeidung von Komplikationen bei der Einnahme werden Nahrungsergänzungsmittel gerne als wässrige Lösung eingenommen. Auch bei Nahrungsmitteln ist die Einnahme in flüssiger Form oft bevorzugt. Um mikrobiellen Befall zu reduzieren und um die chemische Stabilität zu erhöhen, werden Nahrungsergänzungsmittel oft als Brausetabletten oder Pulver angeboten, die dann durch den Anwender in Wasser dispergiert werden, um eine trinkbare Lösung herzustellen.

Unzureichend ist in vielen Fällen das Lösungsverhalten der Inhaltsstoffe der Brausetabletten oder des Pulvers und das optische Erscheinungsbild und der Geschmack der hergestellten Lösung. Unbefriedigend ist bei einigen Nahrungsergänzungsmitteln auch eine ungenügende Bioverfügbarkeit wertvoller Inhaltsstoffe. Nachteilig ist auch, dass viele Nahrungsergänzungsmittel gar nicht auf die speziellen Bedürfnisse bestimmter Konsumenten ausgerichtet sind. Einige Nahrungsergänzungsmittel enthalten eine Vielzahl an Inhaltsstoffen. Somit nehmen einige Konsumenten Inhaltsstoffe zu sich, die sie gar nicht benötigen. Andere Inhaltsstoffe sind nur für spezielle Zielgruppen wichtig und werden den meisten Nahrungsergänzungsmitteln nicht zugesetzt, um Überdosierungen bei Konsumenten, die diese Inhaltsstoffe nicht oder in geringerer Menge benötigen, zu vermeiden oder um ein für möglichst viele Personen erschwingliches Produkt anbieten zu können.

Ferner ist es wünschenswert, die chemische und mikrobielle Stabilität konventioneller Nahrungsergänzungsmittel und Nahrungsmittel zu verbessern.

Es besteht also ein Bedarf an verbesserten Nahrungsergänzungsmitteln und Nahrungsmitteln, die vielseitig und nach individuellem Bedarf einsetzbar sind.

Insbesondere besteht ein Bedarf an Nahrungsergänzungsmitteln, die speziell auf die Bedürfnisse von Menschen ab dem etwa 50. Lebensjahr ausgerichtet sind und die nach individuellem Bedarf und leicht dosierbar gemeinsam mit einem anderen Nahrungsergänzungsmittel oder Nahrungsmittel als Lösung eingenommen werden können.

Als Nahrungsergänzungsmittel oder Nahrungsmittel wünschenswert wäre demnach eine Zusammensetzung, die sich möglichst schnell und vollständig in Wasser löst, um eine gesunde, trinkbare Lösung mit ansprechendem Aussehen und gutem Geschmack zu bilden, die eine hohe Bioverfügbarkeit der Inhaltsstoffe ermöglicht, die insbesondere Personen ab dem etwa 50. Lebensjahr mit den für sie besonders wichtigen Inhaltsstoffen versorgt und die nach individuellem Bedarf und leicht dosierbar gemeinsam mit einem anderen Nahrungsergänzungsmittel oder Nahrungsmittel als Lösung eingenommen werden kann.

Die vorliegende Erfindung richtet sich auf diesen Bedarf und soll die oben aufgeführten Nachteile des Standes der Technik überwinden und löst zumindest einen Teil der Aufgaben durch eine pulverförmige Zusammensetzung, die pro 100 g der Zusammensetzung 25 mg bis 250 mg Lutein, 2 mg bis 12 mg Zeaxanthin, 1000 mg bis 5000 mg Docosahexaensäure, 100 mg bis 1200 mg Coenzym Q10 und 30 g bis 90 g eines Hilfsstoffes oder mehrerer Hilfsstoffe, ausgewählt aus Verdickungsmitteln, Emulgatoren und Stabilisatoren, enthält.

Lutein ist ein lipophiles Xanthophyll, für dessen Verabreichung bei altersbedingter Maculadegeneration, Katarakt und trockenen Augen positive Effekte gezeigt wurden. Auch auf die kognitiven Funktionen und die Gehirnfunktion weist Lutein einen positiven Effekt auf. Vorzugsweise wird das Lutein der Zusammensetzung in Form eines Pulvers zugegeben, das ferner Zeaxanthin, Stärke und getrocknetes Glucosesirup enthält, wobei sich die genannten Mengenangaben in mg auf Lutein in Reinform beziehen. Das Lutein kann der Zusammensetzung auch in Reinform zugegeben werden.

Zeaxanthin ist ebenfalls ein lipophiles Xanthophyll, das zur Prävention der altersbedingten Maculadegeneration geeignet ist. Vorzugsweise wird das Zeaxanthin der Zusammensetzung in Form eines Pulvers zugegeben, das ferner Lutein, Stärke und getrocknetes Glucosesirup enthält, wobei sich die genannten Mengenangaben in mg auf Zeaxanthin in Reinform beziehen. Das Zeaxanthin kann der Zusammensetzung auch in Reinform zugegeben werden.

Lutein und Zeaxanthin scheinen eine besonders wichtige Rolle beim Schutz der Netzhaut und Sehkraft zu spielen. Insbesondere scheinen sie Schäden, die durch blaues Licht (d.h. Licht einer Wellenlänge zwischen 400 und 500 nm) verursacht werden, entgegenzuwirken. Solch blaues Licht wird natürlich von der Sonne, aber auch von LEDs und/oder den Bildschirmen von Fernsehern, Computern und Mobiltelephonen emittiert.

Die ungesättigte Omega-3-Fettsäure Docosahexaensäure hat zahlreiche positive Wirkungen, insbesondere auf das Herz-Kreislaufsystem, das Immunsystem und das Zentralnervensystem. Die Zusammensetzung enthält eine ungesättigte Omega-3-Fettsäure (Docosahexaensäure) oder mehrere ungesättigte Omega-3-Fettsäuren (z.B. Docosahexaensäure und Eicosapentaensäure), die der Zusammensetzung vorzugsweise in Form eines Pulvers zugegeben werden, wobei es sich bei dem Pulver um ein sprühgetrocknetes Pulver handelt, das raffiniertes Algenöl (z.B. aus Schizochytrium sp.), Maissirupfeststoffe und Stärke enthält, wobei sich die genannten Mengenangaben in mg auf Docosahexaensäure in Reinform beziehen. Durch die spezielle Formulierung des Algenöls bzw. der ungesättigte(n) Fettsäure(n) kann eine pulverförmige, rieselfähige und somit gut dosierbare Zusammensetzung erhalten werden.

Coenzym Q10 ist eine fettlösliche Verbindung mit im Allgemeinen schlechter Bioverfügbarkeit. Die Verbindung ist wichtig für den Energiehaushalt und als Radikalfänger. Eine ausreichende Versorgung mit Coenzym Q10 hat positive Effekte auf das Herz, den natürlichen Alterungsprozess und die physische Leistungsfähigkeit. Vorzugsweise wird das Coenzym Q10 der erfindungsgemäßen Zusammensetzung in Form eines Komplexes aus y-Cyclodextrin und Coenzym Q10 (Gewichtsverhältnis im Komplex circa 3:1) zugegeben, wobei sich die Mengenangaben in mg auf das Coenzym Q10 in Reinform beziehen. Durch die Komplexierung kann die Wasserlöslichkeit und die Bioverfügbarkeit des Coenzym Q10 gesteigert werden.

Der eine Hilfsstoff oder die mehreren Hilfsstoffe begünstigen, dass sich die Zusammensetzung schnell und vollständig in Wasser löst, um eine trinkbare Lösung mit ansprechendem Aussehen und gutem Geschmack bereitzustellen. Ferner ermöglicht der Hilfsstoff oder die mehreren Hilfsstoffe, dass die Zusammensetzung pulverförmig ist, so dass die pulverförmige Zusammensetzung nach individuellem Bedarf leicht dosierbar ist und gemeinsam mit einem anderen Nahrungsergänzungsmittel als Lösung eingenommen werden kann.

Durch bloßes Hinzugeben der pulverförmigen Zusammensetzung zu Wasser oder einer wässrigen Lösung in einem Trinkgefäß und kurzes Umrühren kann bei Raumtemperatur (ca. 20 °C) eine Lösung oder zumindest eine trinkbare Dispersion hergestellt werden. Eine Suspension gilt als Lösung bzw. trinkbare Dispersion im Sinne der Erfindung, solange die suspendierten Teile trinkbar dispergiert sind, also im Wesentlichen mitgetrunken werden und nicht am Boden des Trinkgefäßes zurückbleiben. Zusammensetzungen, bei denen die Bestandteile im Wesentlichen auf den Boden des Trinkgefäßes sedimentieren und nicht einfach mitgetrunken werden, gelten nicht als Lösung bzw. trinkbare Dispersion im Sinne der Erfindung. Vorteilhaft an der Formulierung als wasserlösliches Pulver ist, dass die Inhaltsstoffe in trockener Form eine hohe Stabilität aufweisen und bei Anwendung aus der Lösung besonders schnell in den Blutkreislauf gelangen können.

Vorteilhaft ist auch, dass etablierte Nahrungsergänzungsmittel und/oder Nahrungsmittel durch die erfindungsgemäße pulverförmige Zusammensetzung individualisiert werden können und somit beispielsweise Personen ab dem etwa 50. Lebensjahr besonders gut und gezielt versorgt werden können.

Bei der erfindungsgemäßen Zusammensetzung handelt es sich um ein Nahrungsergänzungsmittel und/oder ein Nahrungsmittel.

Die Verben "enthalten" und "umfassen" und ihre Konjugation umfassen auch das Verb "bestehen aus" und seine Konjugationen.

Bevorzugte Ausführungsformen der Erfindung können miteinander kombiniert werden, solange sich aus dem Kontext nichts Anderes ergibt. Bevorzugte Ausführungsformen sind auch in den Ansprüchen angegeben.

Vorzugsweise ist der eine Hilfsstoff oder sind die mehreren Hilfsstoffe ausgewählt aus Gummi arabicum, Alginsäure, Alginaten, Agar-Agar, Carrageenan, Johannisbrotkernmehl, Guarkernmehl, Tragant, Xanthan, Karaya-Gummi, Taragummi, Gellan, Stärken, Stärkephosphaten und Gelatine. Gummi arabicum ist besonders bevorzugt.

Vorzugsweise enthält die Zusammensetzung als den einen oder die mehreren Hilfsstoffe, ausgewählt aus Verdickungsmitteln, Emulgatoren und Stabilisatoren, Gummi arabicum. Mit anderen Worten, besonders bevorzugt enthält die pulverförmige Zusammensetzung pro 100 g der Zusammensetzung 25 mg bis 250 mg Lutein, 2 mg bis 12 mg Zeaxanthin, 1000 mg bis 5000 mg Docosahexaensäure, 100 mg bis 1200 mg Coenzym Q10 und 30 g bis 90 g, bevorzugter 40 g bis 80 g, besonders bevorzugt 50 g bis 70 g, Gummi arabicum.

Gummi arabicum ist besonders geeignet, der pulverförmigen Zusammensetzung das bereits beschrieben Lösungsverhalten und die Trinkbarkeit zu vermitteln. Insbesondere wirkt besonders das Gummi arabicum zugleich als Verdickungsmittel, Emulgator und Stabilisator. Im Ergebnis lassen sich auch lipophile Stoffe lösen bzw. trinkbar dispergieren und einnehmen. Ferner kann gerade mit Gummi arabicum ein Pulver mit guten Fließeigenschaften und somit guter Dosierbarkeit bereitgestellt werden.

Vorzugsweise ist die Zusammensetzung also wasserlöslich oder zumindest lassen sich alle Bestandteile in Wasser (trinkbar) dispergieren (ohne nennenswerte Sedimentation).

Vorzugsweise enthält die Zusammensetzung pro 100 g der Zusammensetzung 1 g bis 10 g, bevorzugter 1 g bis 6 g, besonders bevorzugt 2 g bis 4 g, Zucker. Mit anderen Worten, der Zuckergehalt der Zusammensetzung beträgt pro 100 g der Zusammensetzung 1 g bis 10 g, bevorzugter 1 g bis 6 g, besonders bevorzugt 2 g bis 4 g. Der Zuckergehalt resultiert aus in Reinform zugegebenem Zucker und/oder aus Zucker, der in Extrakten (z.B. Ginsengextrakt) und/oder als Trägerstoff (z.B. für Lutein, Omega-3-Fettsäuren), enthalten ist, und der Zusammensetzung mit dem Extrakt (z.B. Ginsengextrakt) oder als Trägerstoff (z.B. für Lutein, Omega-3-Fettsäuren) zugegeben wird. Der Zuckergehalt führt zu einem guten Geschmack und der Zucker ist selbst wasserlöslich.

Vorzugsweise enthält die Zusammensetzung mindestens ein Steviolglykosid, wodurch der Geschmack verbessert werden kann. Mit anderen Worten, vorzugsweise enthält die Zusammensetzung pro 100 g der Zusammensetzung 0,01 g bis 1 g, bevorzugter 0,1 g bis 0,6 g, besonders bevorzugt 0,2 bis 0,4 g, eines oder mehrerer Steviolglykoside. Durch die Kombination von Zucker und dem mindestens einen Steviolglykosid kann ein besonders gesundes Produkt erhalten werden, da der Zuckergehalt der Zusammensetzung verringert werden kann, die Lösung dennoch Zucker als Energieträger oder Cosubstrat für Transporter im Gastrointestinalsystem enthält.

Vorzugsweise enthält die Zusammensetzung pro 100 g der Zusammensetzung 0,1 g bis 10 g oder 0,3 g bis 7 g oder 0,4 g bis 6 g Extrakt aus rotem Ginseng (Panax ginseng), wobei der Extrakt mindestens 5 Gew.-% Ginsenoside enthält. Vorzugsweise handelt es sich bei dem Extrakt um einen Extrakt aus der Wurzel. Vorzugsweise ist das Extraktionsmittel Wasser. Vorzugsweise wird der Extrakt der Zusammensetzung in Form eines sprühgetrockneten Pulvers mit Maltodextringrundlage zugegeben.

Vorzugsweise enthält die Zusammensetzung pro 100 g der Zusammensetzung 0,5 g bis 5 g, bevorzugter 1 g bis 3 g, Säurungsmittel, vorzugsweise Citronensäure. Das Säurungsmittel, insbesondere die Citronensäure, kann ebenfalls zu dem besonderen Geschmackserlebnis beitragen, in wässriger Lösung der Oxidation bestimmter Inhaltsstoffe entgegenwirken und die Löslichkeit bestimmter Inhaltsstoffe in wässriger Lösung verbessern.

Künstliche Süßstoffe sind nicht erforderlich und in einer bevorzugten Ausführungsform enthält die Zusammensetzung keine künstlichen Süßstoffe. Als künstliche Süßstoffe im Sinne der Erfindung gelten insbesondere Acesulfam (E 950), Advantam (E 969), Aspartam (E 951), Aspartam-Acesulfam-Salz (E 962), Cyclamat (E 952), Neohesperidin (E 959), Neotam (E 961), Saccharin (E 954) und Sucralose (E 955). Steviolglycoside, z.B. Steviosid (E 960), gelten nicht als künstliche Süßstoffe im Sinne der Erfindung.

Die Zusammensetzung ist mikrobiell stabil. Künstliche Konservierungsmittel sind nicht erforderlich und vorzugsweise enthält die Zusammensetzung keine künstlichen Konservierungsmittel.

Vorzugsweise enthält die Zusammensetzung pro 100 g der Zusammensetzung 25 mg bis 200 mg, bevorzugter 40 mg bis 130 mg, besonders bevorzugt 60 mg bis 90 mg, Eicosapentaensäure. Wie die Docosahexaensäure weist auch die ungesättigte Omega-3-Fettsäure Eicosapentaensäure zahlreiche positive Wirkungen auf, insbesondere auf das Herz-Kreislaufsystem, das Immunsystem sowie das Zentralnervensystem.

Vorzugsweise enthält die Zusammensetzung pro 100 g der Zusammensetzung 150 mg bis 3000 mg, bevorzugter 500 mg bis 1500 mg, besonders bevorzugt 750 mg bis 1250 mg, an Isoflavonen, vorzugsweise an Soyaisoflavonen. Isoflavone und ganz besonders Isoflavone aus Soya (Soyaisoflavone) werden bei Wechseljahrsbeschwerden, Osteoporose, Bluthochdruck, hohen Cholesterinwerten, Gedächtnisproblemen, Inkontinenz und einer überaktiven Blase eingesetzt. Soyaisoflavone binden an und transaktivieren bevorzugt den Östrogenrezeptor β und nicht den Östrogenrezeptor α, wodurch sie die Effekte von Östrogen in einigen Geweben nachahmen und die Effekte von Östrogen in anderen Geweben antagonisieren. Östrogenrezeptor α ist der vorherrschende Rezeptor in der Brust und Gebärmutter und Östrogenrezeptor β ist der vorherrschende Rezeptor im Herz-Kreislauf-System, im Urogenitaltrakt und in den Knochen. Dadurch könnten antiöstrogene Wirkungen im Fortpflanzungsgewebe ermöglicht und das Risiko der Entwicklung hormonassoziierter Krebsarten in Brust und Prostata verringert werden. Im Gegensatz dazu kann die östrogene Wirkung in anderen Geweben dazu beitragen, die Knochendichte zu erhalten und die Blutfettprofile zu verbessern. Vorzugsweise werden die Isoflavone aus Soyakeimen, vorzugsweise mittels Ethanol, extrahiert. Soyaisoflavone umfassen insbesondere die Glykoside Genistin, Daidzin und Glycitin sowie deren Aglykone Genistein, Daidzein und Glycitein. Mit anderen Worten, die Zusammensetzung enthält vorzugsweise 150 mg bis 3000 mg, bevorzugter 500 mg bis 1500 mg, besonders bevorzugt 750 mg bis 1250 mg, eines oder mehrerer Isoflavone ausgewählt aus Genistin, Daidzin und Glycitin sowie Genistein, Daidzein und Glycitein. Die Aglykone Genistein, Daidzein und Glycitein sind in einer Ausführungsform bevorzugt, da die Absorption schneller erfolgt und die Bioverfügbarkeit höher ist.

Darmbakterien können Glykoside in Aglykone umwandeln und so die Absorption im Gastrointestinaltrakt verbessern. Darmbakterien können außerdem Daidzein in Equol umwandeln. Equol weist an dem Östrogenrezeptor β eine größere östrogene Aktivität als Daidzein auf, weshalb davon ausgegangen wird, dass Equol einige, wenn nicht sogar die meisten gesundheitlichen Vorteile der Isoflavone vermittelt. Equol [7-Hydroxy-3-(4'-Hydroxyphenyl)-Chroman] ist ein nichtsteroidales Östrogen. Es weist die Summenformel C15H₁₄O₃ und ein Molekulargewicht von 242,27 Dalton auf. Studien, die die Ausscheidung von Equol im Urin nach Sojakonsum messen, zeigen, dass Equol von etwa 25 % bis 30 % der erwachsenen Bevölkerung in den westlichen Ländern produziert wird. Im Gegensatz dazu produzieren 50 % bis 60 % der Erwachsenen, die in asiatischen Ländern leben und/oder in westlichen Ländern leben und sich vegetarisch ernähren, Equol. Verschiedene in vitro kultivierte Darmbakterien können Daidzein zu S-(-)Equol oder verwandten Zwischenprodukten biotransformieren. Dies steht im Einklang mit der Beobachtung, dass Equolproduzenten eine höhere Komplexität des Darmmikrobioms aufweisen. Im Gegensatz dazu scheint der Einsatz von Antibiotika die Aglykonproduktion zu verringern. Die Einnahme von Soyaisoflavonen über einen Zeitraum von mehr als 16 Wochen kann das Wachstum von Darmbakterien, die die Equolproduktion unterstützen, positiv fördern.

Vorzugsweise enthält die Zusammensetzung pro 100 g der Zusammensetzung 1 g bis 10 g, bevorzugter 1 g bis 6 g, besonders bevorzugt 2 g bis 4 g, gesättigte Fettsäuren. Somit ist das Produkt arm an gesättigten Fettsäuren und besonders gesund.

Vorzugsweise enthält die Zusammensetzung pro 100 g der Zusammensetzung 50 mg bis 175 mg, besonders bevorzugt 75 mg bis 125 mg, Lutein.

Vorzugsweise enthält die Zusammensetzung pro 100 g der Zusammensetzung 3 mg bis 10 mg, besonders bevorzugt 5 mg bis 7 mg, Zeaxanthin.

Vorzugsweise enthält die Zusammensetzung pro 100 g der Zusammensetzung 1500 mg bis 3500 mg, besonders bevorzugt 2000 mg bis 3000 mg, Docosahexaensäure.

Vorzugsweise enthält die Zusammensetzung pro 100 g der Zusammensetzung 300 mg bis 1000 mg, besonders bevorzugt 500 mg bis 700 mg, Coenzym Q10.

Gegenstand der Erfindung ist auch Kit, enthaltend eine erfindungsgemäße pulverförmige Zusammensetzung ("erste Zusammensetzung") und eine zweite pulverförmige Zusammensetzung, wobei die zweite pulverförmige Zusammensetzung ein Nahrungsergänzungsmittel und/oder Nahrungsmittel ist. In dem Kit liegen die erste und zweite Zusammensetzung räumlich getrennt voneinander vor, beispielsweise sind die erste und zweite Zusammensetzung innerhalb des Kits jeweils in einem Behältnis verpackt. Vorteilhaft ist, dass etablierte Nahrungsergänzungsmittel und/oder Nahrungsmittel durch die erfindungsgemäße pulverförmige Zusammensetzung individualisiert werden können und somit beispielsweise Personen ab dem etwa 50. Lebensjahr besonders gut und gezielt versorgt werden können.

Gegenstand der Erfindung ist auch die Verwendung der erfindungsgemäßen Zusammensetzung oder des erfindungsgemäßen Kits als Nahrungsergänzungsmittel und/oder Nahrungsmittel, wobei von der (ersten) Zusammensetzung an einem Tag 1 g bis 10 g, bevorzugter 2 g bis 8 g, besonders bevorzugt 4 g bis 6 g, eingenommen werden. Die Zusammensetzung wird vorzugsweise von einer Person eingenommen, die mindestens 50 Jahre alt ist. Vorzugsweise wird die Zusammensetzung oral als Lösung bzw. trinkbare Dispersion eingenommen. In einer bevorzugten Ausführungsform ist die Verwendung keine medizinische Verwendung.

Gegenstand der Erfindung ist auch ein Verfahren zur Herstellung einer Lösung zur oralen Applikation. Hierbei wird die erfindungsgemäße pulverförmige Zusammensetzung und gegebenenfalls eine zweite pulverförmige Zusammensetzung, die ein Nahrungsergänzungsmittel und/oder Nahrungsmittel ist, in Wasser gelöst bzw. dispergiert, um eine trinkbare Lösung herzustellen. Dabei werden 3 g bis 7 g, vorzugsweise 4 g bis 6 g, der erfindungsgemäßen (ersten) Zusammensetzung in circa 100 mL bis 300 mL, vorzugsweise 150 mL bis 250 mL, Wasser gegeben und gemischt, so dass eine Lösung oder zumindest eine trinkbare Dispersion erhalten wird. Vorzugsweise erfolgt das Lösen bei 10 °C bis 30 °C, besonders bevorzugt bei 15 °C bis 25 °C oder circa bei Raumtemperatur.

Gegenstand der Erfindung ist auch ein Verfahren zur Herstellung einer erfindungsgemäßen Zusammensetzung, umfassend das Mischen von 25 mg bis 250 mg Lutein, 2 mg bis 12 mg Zeaxanthin, 1000 mg bis 5000 mg Docosahexaensäure, 100 mg bis 1200 mg Coenzym Q10 und 30 g bis 90 g eines oder mehrerer Hilfsstoffe, ausgewählt aus Verdickungsmitteln, Emulgatoren und Stabilisatoren, sowie gegebenenfalls eines oder mehrerer Inhaltsstoffe, ausgewählt aus 0,1 g bis 10 g Extrakt aus rotem Ginseng (Panax ginseng), 0,5 g bis 5 g Säurungsmittel, vorzugsweise Citronensäure, 25 mg bis 200 mg Eicosapentaensäure, 150 mg bis 3000 mg, an Isoflavonen, vorzugsweise an Soyaisoflavonen, und 0,01 g bis 1 g eines oder mehrerer Steviolglykoside.

In einer bevorzugten Ausführungsform des Kits, enthält das Kit als zweite pulverförmige Zusammensetzung das nachfolgend beschriebene Nahrungsergänzungsmittel bzw. Nahrungsmittel. Die nachfolgenden Ausführungen bis zum Beispielteil gelten ausschließlich für die zweite Zusammensetzung.

Die zweite pulverförmige Zusammensetzung ist eine pulverförmige Zusammensetzung zur Herstellung einer Lösung zur oralen Applikation, wobei die Zusammensetzung pro 100 g, 1 g bis 85 g mindestens eines Monosaccharides, 0,18 g bis 0,70 g mindestens eines Steviolglykosides, 1,5 g bis 10 g Citronensäure, 0,3 g bis 3 g Vitamin C, mindestens zwei weitere Vitamine und einen Extrakt aus grünem Tee (Camellia sinensis) enthält. Vorzugsweise löst sich die zweite pulverförmige Zusammensetzung möglichst schnell und vollständig in Wasser, um eine trinkbare Lösung bereitzustellen, wobei die Lösung ein ansprechendes Aussehen und einen guten Geschmack aufweist. Vorzugsweise ermöglicht die zweite pulverförmige Zusammensetzung eine hohe Bioverfügbarkeit der Inhaltsstoffe und/oder verbessert die Sauerstoffversorgung im menschlichen Körper und/oder reduziert oxidativen Stress und/oder erhöht die Konzentration an körpereigenem Stickstoffmonoxid.

Das mindestens eine Monosaccharid führt zu der schnellen Lösung des Pulvers in Wasser und zu einem guten Geschmack. Das mindestens eine Steviolglykosid trägt ebenfalls zu dem guten Geschmack bei. Durch die Kombination von mindestens einem Monosaccharid und mindestens einem Steviolglykosid wird ein besonders gesundes Produkt erhalten, da der Anteil an Monosacchariden verringert werden kann und die Lösung dennoch mindestens ein Monosaccharid als Energieträger enthält. Die Citronensäure trägt ebenfalls zu dem besonderen Geschmackserlebnis bei. Künstliche Süßstoffe sind nicht erforderlich und vorzugsweise enthält die zweite Zusammensetzung keine künstlichen Süßstoffe.

Die zweite Zusammensetzung ist mikrobiell stabil. Künstliche Konservierungsmittel sind nicht erforderlich und vorzugsweise enthält die zweite Zusammensetzung keine künstlichen Konservierungsmittel.

Bei dem Vitamin C und den mindestens zwei weiteren Vitaminen handelt es sich um Vitamine, die nicht Bestandteil des Extraktes aus grünem Tee sind. Vorzugsweise ist das Vitamin C und sind die mindestens zwei weiteren Vitamine als Reinstoff beigefügt.

In bevorzugten Ausführungsformen enthält die zweite Zusammensetzung pro 100 g 5 g bis 80 g des mindestens einen Monosaccharides, 0,19 g bis 0,60 g des mindestens einen Steviolglykosides, 2,5 g bis 7,0 g Citronensäure und 0,4 g bis 2,5 g Vitamin C.

Vorzugsweise enthält die zweite Zusammensetzung pro 100 g 4,14 mg bis 3000 mg des Extraktes aus grünem Tee (Camellia sinensis), vorzugsweise aus Blättern des grünen Tees. Der Extrakt enthält wertvolle Polyphenole, Catechine und trägt zu dem besonderen Geschmack der Lösung bei.

Vorzugsweise enthält die zweite Zusammensetzung
ein Konzentrat aus dem Broccoli (Brassica oleracea italica), insbesondere aus Samen, Keimen und/oder Sprossen des Broccolis, und/oder
ein Konzentrat aus dem Broccoli (Brassica oleracea italica), insbesondere aus Röschen und/oder Stielen des Broccolis, und/oder
einen Extrakt aus der Zwiebel (Allium cepa, insbesondere Allium cepa alliaceae), insbesondere aus der Zwiebel der Zwiebel, und/oder
einen Extrakt aus dem Apfel (Malus domestica), insbesondere aus der Apfelfrucht, insbesondere aus Schalen der Apfelfrucht, und/oder
einen Extrakt aus der Acerola (Malpighia glabra, insbesondere Malpighia glabra linne), insbesondere aus der Frucht der Acerola, und/oder
ein Konzentrat aus der Tomate (Lycopersicon esculentum), insbesondere aus der Frucht der Tomate, und/oder
ein Konzentrat aus der Kurkuma (Curcuma longa), insbesondere aus der Wurzel der Kurkuma, und/oder
ein Konzentrat aus dem Knoblauch (Allium sativum), insbesondere aus der Knoblauchzehe, und/oder
ein Konzentrat aus dem Basilikum (Ocimum basilicum), insbesondere aus Blättern des Basilikums, und/oder
ein Konzentrat aus dem Oregano (Origanum vulgare), insbesondere aus Blättern des Oreganos, und/oder
ein Konzentrat aus dem Zimtbaum (Cinnamonum cassia), insbesondere aus der Rinde (ohne Kork) des Zimtbaumes, und/oder
ein Konzentrat aus der Karotte (Dacus carota, insbesondere Dacus carota sativa), insbesondere aus der Wurzel der Karotte, und/oder
ein Konzentrat aus dem Holunder (Sambucus nigra), insbesondere aus der Frucht des Holunders, und/oder
einen Extrakt aus der Johannisbeere (Ribes), insbesondere der schwarzen Johannisbeere (Ribes nigrum), insbesondere aus der Frucht der schwarzen Johannisbeere, und/oder
ein Konzentrat aus der Heidelbeere (Vaccinium), insbesondere aus der Frucht der Heidelbeere, und/oder
ein Konzentrat aus der Himbeere (Rubus idaeus), insbesondere aus der Frucht der Himbeere, und/oder
ein Konzentrat aus der Brombeere (Rubus spp.), insbesondere aus der Frucht der Brombeere, und/oder
ein Konzentrat aus der Apfelbeere (Aronia, insbesondere Aronia melanocarpa), insbesondere aus der Frucht der Apfelbeere, und/oder
ein Konzentrat aus dem Spinat (Spinacia oleracea), insbesondere aus den Blättern des Spinates, und/oder
ein Konzentrat aus der Kirsche (Prunus avium), insbesondere aus der Kirschfrucht, und/oder
ein Konzentrat aus dem Rosenkohl (Brassica oleracea gemmifera), insbesondere aus Röschen des Rosenkohles.

Vorzugsweise enthält die zweite Zusammensetzung pro 100 g der Zusammensetzung von diesen Extrakten und/oder Konzentraten insgesamt 0,10 g bis 0,75 g.

Vorzugsweise enthält die zweite Zusammensetzung
ein Konzentrat aus dem Broccoli (Brassica oleracea italica), insbesondere aus Samen, Keimen und/oder Sprossen des Broccolis, und
ein Konzentrat aus dem Broccoli (Brassica oleracea italica), insbesondere aus Röschen und/oder Stielen des Broccolis, und
einen Extrakt aus der Zwiebel (Allium cepa, insbesondere Allium cepa alliaceae), insbesondere aus der Zwiebel der Zwiebel, und
einen Extrakt aus dem Apfel (Malus domestica), insbesondere aus der Apfelfrucht, insbesondere aus Schalen der Apfelfrucht, und
einen Extrakt aus der Acerola (Malpighia glabra, insbesondere Malpighia glabra linne), insbesondere aus der Frucht der Acerola, und
ein Konzentrat aus der Tomate (Lycopersicon esculentum), insbesondere aus der Frucht der Tomate, und
ein Konzentrat aus der Kurkuma (Curcuma longa), insbesondere aus der Wurzel der Kurkuma, und
ein Konzentrat aus dem Knoblauch (Allium sativum), insbesondere aus der Knoblauchzehe, und
ein Konzentrat aus dem Basilikum (Ocimum basilicum), insbesondere aus Blättern des Basilikums, und
ein Konzentrat aus dem Oregano (Origanum vulgare), insbesondere aus Blättern des Oreganos, und
ein Konzentrat aus dem Zimtbaum (Cinnamonum cassia), insbesondere aus der Rinde (ohne Kork) des Zimtbaumes, und
ein Konzentrat aus der Karotte (Dacus carota, insbesondere Dacus carota sativa), insbesondere aus der Wurzel der Karotte, und
ein Konzentrat aus dem Holunder (Sambucus nigra), insbesondere aus der Frucht des Holunders, und
einen Extrakt aus der Johannisbeere (Ribes), insbesondere der schwarzen Johannisbeere (Ribes nigrum), insbesondere aus der Frucht der schwarzen Johannisbeere, und
ein Konzentrat aus der Heidelbeere (Vaccinium), insbesondere aus der Frucht der Heidelbeere, und
ein Konzentrat aus der Himbeere (Rubus idaeus), insbesondere aus der Frucht der Himbeere, und
ein Konzentrat aus der Brombeere (Rubus spp.), insbesondere aus der Frucht der Brombeere, und
ein Konzentrat aus der Apfelbeere (Aronia, insbesondere Aronia melanocarpa), insbesondere aus der Frucht der Apfelbeere, und
ein Konzentrat aus dem Spinat (Spinacia oleracea), insbesondere aus den Blättern des Spinates, und
ein Konzentrat aus der Kirsche (Prunus avium), insbesondere aus der Kirschfrucht, und
ein Konzentrat aus dem Rosenkohl (Brassica oleracea gemmifera), insbesondere aus Röschen des Rosenkohles.

Vorzugsweise enthält die zweite Zusammensetzung pro 100 g der Zusammensetzung von diesen Extrakten und Konzentraten insgesamt 0,10 g bis 0,75 g.

Der Begriff "Extrakt" bezeichnet im Bezug auf die zweite Ausführungsform einen eingetrockneten Auszug aus entsprechenden Pflanzenteilen, wobei der Extrakt bevorzugt mit Hilfe eines organischen Lösungsmittels und/oder Wasser und mit einem Droge-Extrakt-Verhältnis zwischen 120:1 und 10:1 gewonnen wird. Ein "Konzentrat" bezeichnet ein entwässertes Produkt aus entsprechenden Pflanzenteilen. Extrakte und Konzentrate aus Früchten, Blättern, Zwiebeln, Rinde (ohne Kork), Wurzeln, Rhizomen, Knollen, Samen, Keimen, Sprossen, Röschen und Stielen bieten je nach Pflanzenart einen hohen Gehalt an wertvollen, pflanzlichen Sekundärmetaboliten wie Polyphenolen. Polyphenole sind aromatische Verbindungen, die zwei oder mehr direkt an einen aromatischen Ring gebundene Hydroxylgruppen enthalten und zu den pflanzlichen Sekundärmetaboliten gerechnet werden. Polyphenole weisen verschiedene gesundheitsfördernde Effekte auf, insbesondere wirken einige Polyphenole antioxidativ. Die Konzentrate und Extrakte verschiedenster Pflanzen stellen eine Vielzahl gesundheitsfördernder pflanzlicher Sekundärmetabolite, Vitamine und Polyphenole bereit. Eine solche zweite Zusammensetzung kann die Bioverfügbarkeit der in der zweiten Zusammensetzung oder einer Lösung, die die erste und die zweite Zusammensetzung enthält, enthaltenen Verbindungen erhöhen. Daher weist die zweite Zusammensetzung eine besonders gesundheitsfördernde Wirkung auf und unterstützt eine ausgewogene Ernährung besonders gut. Ferner wurde herausgefunden, dass sich die Beigabe der Extrakte und/oder Konzentrate positiv auf das Löseverhalten des Pulvers in Wasser und den Geschmack der Lösung auswirkt.

Vorzugsweise enthält die zweite Zusammensetzung pro 100 g jeweils 4 mg bis 200 mg, bevorzugt 5 mg bis 50 mg, ein oder mehrerer Inhaltsstoffe ausgewählt aus einem Extrakt aus dem Pfeffer (Piper nigrum) und einem Extrakt aus dem Ingwer (Zingiber officinale). Vorzugsweise enthält die zweite Zusammensetzung pro 100 g 4 mg bis 200 mg, bevorzugt 5 mg bis 50 mg, eines Extraktes aus dem Pfeffer (Piper nigrum), 0,25 mg bis 2 g, bevorzugt 0,45 mg bis 1,5 g, einer Curcuminverbindung und 4 mg bis 200 mg, bevorzugt 5 mg bis 50 mg, eines Extraktes aus dem Ingwer (Zingiber officinale). Bei der Curcuminverbindung kann es sich um einen Curcumin-Cyclodextrin-Komplex, insbesondere einen Curcumin-Gamma-Cyclodextrin-Komplex, handeln. Bevorzugt handelt es sich bei dem Extrakt aus dem Ingwer um einen Extrakt aus dem Rhizom des Ingwers (Zingiber officinale). Diese Substanzen wirken, auch in so geringer Dosierung, als Bioenhancer und steigern die Bioverfügbarkeit der Inhaltsstoffe der Zusammensetzung. Die Verbesserung der Bioverfügbarkeit kann durch das Lösen der ersten und zweiten Zusammensetzung in einem Wasserglas auch für Inhaltsstoffe ausgenutzt werden, die nur in der ersten Zusammensetzung enthalten sind. Somit kann die zweite Zusammensetzung die Bioverfügbarkeit der ersten Zusammensetzung steigern.

Vorzugsweise enthält die zweite Zusammensetzung pro 100 g 2,3 mg bis 5 g, bevorzugter 4,14 mg bis 3 g, Extrakt aus dem Kaffee (Coffea arabica), vorzugsweise aus der Bohne des Kaffees.

Vorzugsweise enthält die zweite Zusammensetzung pro 100 g 0,025 mg bis 2 g, bevorzugter 0,045 mg bis 1,5 g, Extrakt aus der Sauerkirsche (Prunus cerasus), vorzugsweise aus der Kirsche der Sauerkirsche.

Vorzugsweise enthält die zweite Zusammensetzung pro 100 g 0,025 mg bis 2 g, bevorzugter 0,045 mg bis 1,5 g Extrakt aus dem Kohl (Brassica oleracea acephala), vorzugsweise aus Blättern des Kohls.

Vorzugsweise enthält die zweite Zusammensetzung pro 100 g 0,25 mg bis 2 g, bevorzugter 0,45 mg bis 1,5 g Extrakt aus der Kurkuma (Curcuma longa), vorzugsweise aus der Wurzel der Kurkuma.

Vorzugsweise enthält die zweite Zusammensetzung pro 100 0,025 mg bis 2 g, bevorzugter 0,045 mg bis 1,5 g Extrakt aus dem Broccoli (Brassica oleracea italica), vorzugsweise aus dem Röschen des Broccolis.

Vorzugsweise enthält die zweite Zusammensetzung pro 100 g 0,025 mg bis 2 g, bevorzugter 0,045 mg bis 1,5 g, Extrakt aus der Heidelbeere (Vaccinium corymbosum), vorzugsweise aus der Frucht der Heidelbeere.

Vorzugsweise enthält die zweite Zusammensetzung einen Extrakt aus dem Kaffee (Coffea arabica), vorzugsweise aus der Bohne des Kaffees, einen Extrakt aus der Sauerkirsche (Prunus cerasus), vorzugsweise aus der Kirsche der Sauerkirsche, einen Extrakt aus dem Kohl (Brassica oleracea acephala), vorzugsweise aus Blättern des Kohls, einen Extrakt aus der Kurkuma (Curcuma longa), vorzugsweise aus der Wurzel der Kurkuma, einen Extrakt aus dem Broccoli (Brassica oleracea italica), vorzugsweise aus dem Röschen des Broccolis, einen Extrakt aus der Heidelbeere (Vaccinium corymbosum), vorzugsweise aus der Frucht der Heidelbeere und den Extrakt aus grünem Tee (Camellia sinensis), vorzugsweise aus Blättern des grünen Tees, wobei die zweite Zusammensetzung von diesen Extrakten pro 100 g insgesamt 0,01 g bis 5 g enthält. Diese Extrakte, insbesondere in Kombination, können die Konzentration an körpereigenem Stickstoffmonoxid erhöhen.

Vorzugsweise enthält die zweite Zusammensetzung pro 100 g der Zusammensetzung 100 mg bis 1000 mg Koffein. Das Koffein kann aus einem Guaranaextrakt stammen.

In einer Ausführungsform der zweiten Zusammensetzung sind die mindestens zwei weiteren Vitamine ausgewählt aus B-Vitaminen (B-Vitamin-Ausführungsform). Vorzugsweise enthält die zweite Zusammensetzung in dieser Ausführungsform pro 100 g der Zusammensetzung 1 g bis 85 g, bevorzugter 5 bis 80 g, des mindestens einen Monosaccharides. Vorzugsweise enthält die zweite Zusammensetzung in dieser Ausführungsform pro 100 g der Zusammensetzung 0,19 g bis 0,60 g des mindestens einen Steviolglykosides. Vorzugsweise enthält die zweite Zusammensetzung in dieser Ausführungsform pro 100 g der Zusammensetzung 2,5 g bis 7,0 g Citronensäure. Vorzugsweise enthält die zweite Zusammensetzung pro 100 g der Zusammensetzung in dieser Ausführungsform ferner 50 mg bis 200 mg Algenpulver und gegebenenfalls 3 g bis 12 g, bevorzugter 4 g bis 10 g, Guaranaextrakt. Der Guaranaextrakt kann unter anderem Koffein beisteuern. Das Algenpulver ist vorzugsweise ein Pulver aus Braunalgen. Vorzugsweise enthält die zweite Zusammensetzung in dieser Ausführungsform Dextrose als das mindestens eine Monosaccharid. In dieser Ausführungsform enthält die zweite Zusammensetzung pro 100 g der Zusammensetzung als B-Vitamine vorzugsweise 100 mg bis 1500 mg Niacin, 20 mg bis 270 mg Pantothensäure, 5 mg bis 100 mg Pyridoxin, 4 mg bis 72 mg Riboflavin, 5 mg bis 70 mg Thiamin, 10 µg bis 10 mg Cyanocobolamin, 0,5 mg bis 7 mg Biotin und 0,5 mg bis 12 mg Folsäure. Dextrose löst sich sehr schnell in Wasser und verleiht der Lösung in Kombination mit den anderen Inhaltsstoffen den besonderen Geschmack. Ferner verbessert Dextrose die Resorption bestimmter Inhaltsstoffe. Diese Ausführungsform hilft insbesondere dabei, die Sauerstoffversorgung der Gewebe zu verbessern.

In einer alternativen Ausführungsform der zweiten Zusammensetzung sind die mindestens zwei weiteren Vitamine ausgewählt aus Vitaminen A und E (Vitamine A und E-Ausführungsform). Vorzugsweise enthält die zweite Zusammensetzung in dieser Ausführungsform pro 100 g der Zusammensetzung 1500 µg bis 4000 µg Retinol-Äquivalente und 60 mg bis 120 mg α-Tocopherol-Äquivalente. Vorzugsweise enthält die zweite Zusammensetzung in dieser Ausführungsform pro 100 g der Zusammensetzung 5 g bis 80 g, bevorzugter 5 g bis 70 g, besonders bevorzugt 5 g bis 42 g, des mindestens einen Monosaccharides. Vorzugsweise enthält die zweite Zusammensetzung in dieser Ausführungsform pro 100 g der Zusammensetzung 0,19 g bis 0,60 g des mindestens einen Steviolglykosides. Vorzugsweise enthält die zweite Zusammensetzung in dieser Ausführungsform pro 100 g der Zusammensetzung 2,0 g bis 7,0 g Citronensäure. Vorzugsweise enthält die zweite Zusammensetzung in dieser Ausführungsform Fructose als das mindestens eine Monosaccharid. Vorzugsweise enthält die zweite Zusammensetzung in dieser Ausführungsform pro 100 g der Zusammensetzung 200 µg bis 350 µg Selen. Vorzugsweise enthält die zweite Zusammensetzung in dieser Ausführungsform pro 100 g der Zusammensetzung 35 g bis 85 g Ballaststoffe. Bei den Ballaststoffen handelt es sich vorzugsweise um pflanzliche Fasern aus Hafer, Erbse, Reis und/oder Apfel. Fructose löst sich sehr schnell in Wasser und verleiht der Lösung in Kombination mit den anderen Inhaltsstoffen den wohltuenden Geschmack. Erstaunlich ist, dass sich mit dieser speziellen zweiten Zusammensetzung trotz des hohen Ballaststoffanteils eine Lösung herstellen lässt. Diese Ausführungsform ist insbesondere förderlich für die Gesundheit des Darmes und stimuliert das Immunsystem. Die Inhaltsstoffe der zweiten (und ersten) Zusammensetzung können in dieser Variante noch besser aufgenommen werden. In dieser alternativen Ausführungsform der zweiten Zusammensetzung ist auch bevorzugt, dass die Zusammensetzung Verdauungsenzyme (Amylase, Protease, Lactase, Cellulase und Lipase) und einen Curcumin-Gamma-Cyclodextrin-Komplex enthält. In dieser alternativen Ausführungsform der zweiten Zusammensetzung ist auch bevorzugt, dass die Zusammensetzung gram-positive nicht-sporenbildende Bakterien, wie z.B. (Lactobacillus acidophilus, Lactobacillus reuteri) und/oder gram positive sporenbildende Bakterien, wie z.B. Bacillus species, enthält.

Die zweite Zusammensetzung kann ferner Hilfsstoffe wie z.B. Rote-Bete-Pulver (Konzentrat des Saftes roter Bete, Maltodextrin), Gummi arabicum, Pectin und/oder Guargummi enthalten.

Mit Blick auf die Verwendung der zweiten Zusammensetzung in einem Kit als Nahrungsergänzungsmittel und/oder Nahrungsmittel ist bevorzugt, dass von der B-Vitamin-Ausführungsform der zweiten Zusammensetzung 1,25 g bis 2,25 g der Zusammensetzung dreimal täglich eingenommen werden oder alternativ von der Vitamine A und E-Ausführungsform der zweiten Zusammensetzung einmal täglich 8 g bis 16 g der Zusammensetzung eingenommen werden. Die Einnahme erfolgt oral, vorzugsweise als Lösung bzw. trinkbare Dispersion.

Mit Blick auf das Verfahren zur Herstellung einer Lösung zur oralen Applikation ist bevorzugt, dass bei der B-Vitamin-Ausführungsform der zweiten Zusammensetzung 1,25 g bis 2,25 g der zweiten Zusammensetzung in 20 mL bis 100 mL, bevorzugt circa 40 mL, Wasser gelöst (bzw. trinkbar dispergiert) werden und bei der Vitamine A und E-Ausführungsform der zweiten Zusammensetzung 8 g bis 16 g der zweiten Zusammensetzung in 100 mL bis 300 mL, bevorzugt circa 180 mL, Wasser gelöst (bzw. trinkbar dispergiert) werden. Vorzugsweise erfolgt das Lösen bei 10 °C bis 30 °C, besonders bevorzugt bei 15 °C bis 25 °C oder circa bei Raumtemperatur. Vorzugsweise wird zunächst die zweite Zusammensetzung in Wasser gelöst und dann die erste Zusammensetzung zugegeben und gelöst.

### Beispiele

Die Erfindung wird nachfolgend an Hand eines Beispieles erläutert.

Eine (erste) erfindungsgemäße pulverförmige Zusammensetzung wird durch Mischen 25 g eines sprühgetrockneten Pulvers, das raffiniertes Algenöl aus Schizochytrium sp., Maissirupfeststoffe und Stärke enthält, 3 g eines Komplexes aus y-Cyclodextrin und Coenzym Q10, 0,9 g eines Extraktes aus rotem Ginseng mit mindestens 5 % Ginsenosiden, 0,3 g Steviolglykoside, 1,5 g Citronensäure, 2 g eines Pulvers, das Lutein, Zeaxanthin, Stärke und getrocknetes Glucosesirup enthält, 2,5 g eines Soyaextraktes mit Isoflavonen und pulverförmigen Gummi arabicums ad 100 g erhalten. 100 g der Zusammensetzung enthalten u.a. die folgenden Inhaltsstoffe:

| | |
|---|---|
| Coenzym Q10 | 660 mg |
| Lutein | 110 mg |
| Zeaxanthin | 6,6 mg |
| Isoflavone | 1050 mg |
| Docosahexaensäure | 2625 mg |
| Eicosapentaensäure | 78 mg |

Die Nährwerttabelle enthält für 100 g der Zusammensetzung folgende Werte:

| | |
|---|---|
| Brennwert | 287 kcal |
| Fettgehalt | 8 g |
| davon qesättiqt | 2,7 g |
| Kohlenhydratgehalt | 26 g |
| davon Zucker | 3 g |

Durch bloßes Hinzugeben der pulverförmigen Zusammensetzung zu Wasser oder einer wässrigen Lösung in einem Trinkgefäß und kurzes Umrühren kann bei Raumtemperatur (ca. 20 °C) eine Lösung oder zumindest eine trinkbare Dispersion hergestellt werden.

Die Lösung weist ein ansprechendes Aussehen und einen guten Geschmack auf, versorgt insbesondere Personen ab dem etwa 50. Lebensjahr mit für sie besonders wichtigen Inhaltsstoffen. Die pulverförmige Zusammensetzung ist rieselfähig, nach individuellem Bedarf und leicht dosierbar und kann gemeinsam mit einem Nahrungsergänzungsmittel oder Nahrungsmittel als Lösung eingenommen werden.

Diese erfindungsgemäße ("erste") Zusammensetzung kann in einem Kit gemeinsam mit einer zweiten Zusammensetzung angeboten werden. Nachfolgend sind zwei Beispielrezepturen für solche zweiten Zusammensetzungen angegeben.

Beispielrezeptur für die zweite Zusammensetzung gemäß B-Vitamin-Ausführungsform der zweiten Zusammensetzung. 100 g der pulverförmigen Zusammensetzung enthalten:
- Vitamin C, 2,5 g;
- Niacin, 0,34 g;
- Koffein, 0,8 g;
- Pantothensäure, 0,05 g;
- Pyridoxin, 0,02 g;
- Thiamin, 0,02 g;
- Riboflavin, 0,02 mg;
- Cyanocobolamin, 10 µg;
- Folsäure, 2,0 mg;
- Biotin, 1,5 mg;
- Citronensäure, 7,5 g;
- Guaranaextrakt, 9,5 g;
- Steviolglykoside, 0,4 g;
- Algenpulver, 0,1 g;
- Extrakt aus grünem Tee (Camellia sinensis), 5 mg;
- Rote-Bete-Pulver (Konzentrat des Saftes roter Bete, Maltodextrin), 1 g;
- Dextrose ad 100 g.

Beispielrezeptur für die zweite Zusammensetzung gemäß Vitamine A und E-Ausführungsform der zweiten Zusammensetzung. 100 g der pulverförmigen Zusammensetzung enthalten:
- Steviolglykoside, 0,3 g;
- Citronensäure, 3,5 g;
- Vitamin C, 1,5 g;
- Extrakt aus grünem Tee (Camellia sinensis), 0,08 g;
- 2000 µg Retinol-Äquivalente;
- 70 mg α-Tocopherol-Äquivalente;
- 250 µg Selen;
- 46 g Ballaststoffe;
- Lactobacillus acidophilus, Lactobacillus reuteri, 0,3 g;
- Amylase, Protease, Lactase, Cellulase und Lipase, 0,3 g;
- Fructose ad 100 g.

Die pulverförmigen zweiten Zusammensetzungen lösen sich schnell in Wasser und bilden trinkbare Lösungen ohne künstliche Konservierungsstoffe oder Süßstoffe. Die Lösungen weisen ein ansprechendes Aussehen und einen guten Geschmack auf, bieten eine hohe Bioverfügbarkeit der Inhaltsstoffe und verbessern die Versorgung des menschlichen Körpers mit Sauerstoff, wichtigen Vitaminen, Polyphenolen und anderen Stoffen. In diesen Lösungen der zweiten Zusammensetzungen lösen sich die erfindungsgemäßen ersten pulverförmigen Zusammensetzungen schnell.

## Patentansprüche

1. Pulverförmige Zusammensetzung, enthaltend pro 100 g der Zusammensetzung
25 mg bis 250 mg Lutein,
2 mg bis 12 mg Zeaxanthin,
1000 mg bis 5000 mg Docosahexaensäure,
100 mg bis 1200 mg Coenzym Q10 und
30 g bis 90 g eines Hilfsstoffes oder mehrerer Hilfsstoffe, ausgewählt aus Verdickungsmitteln, Emulgatoren und Stabilisatoren.

2. Zusammensetzung nach Anspruch 1, **dadurch gekennzeichnet, dass** die ein oder mehreren Hilfsstoffe ausgewählt sind aus Gummi arabicum, Alginsäure, Alginaten, Agar-Agar, Carrageenan, Johannisbrotkernmehl, Guarkernmehl, Tragant, Xanthan, Karaya-Gummi, Taragummi, Gellan, Stärken, Stärkephosphaten und Gelatine.

3. Zusammensetzung nach Anspruch 1 oder 2, **dadurch gekennzeichnet, dass** die Zusammensetzung pro 100 g der Zusammensetzung als Hilfsstoff 30 g bis 90 g, bevorzugter 40 g bis 80 g, besonders bevorzugt 50 g bis 70 g, Gummi arabicum enthält.

4. Zusammensetzung nach irgendeinem der vorherigen Ansprüche, **dadurch gekennzeichnet, dass** die Zusammensetzung wasserlöslich ist.

5. Zusammensetzung nach irgendeinem der vorherigen Ansprüche, **dadurch gekennzeichnet, dass** die Zusammensetzung pro 100 g der Zusammensetzung 1 g bis 10 g, bevorzugter 1 g bis 6 g, besonders bevorzugt 2 g bis 4 g, Zucker enthält.

6. Zusammensetzung nach irgendeinem der vorherigen Ansprüche, **dadurch gekennzeichnet, dass** die Zusammensetzung pro 100 g der Zusammensetzung 0,1 g bis 10 g, bevorzugter 0,3 g bis 7 g, besonders bevorzugt 0,4 g bis 6 g, Extrakt aus rotem Ginseng (Panax ginseng) enthält.

7. Zusammensetzung nach irgendeinem der vorherigen Ansprüche, **dadurch gekennzeichnet, dass** die Zusammensetzung pro 100 g der Zusammensetzung 0,5 g bis 5 g, bevorzugter 1 g bis 3 g, Säurungsmittel, vorzugsweise Citronensäure, enthält.

8. Zusammensetzung nach irgendeinem der vorherigen Ansprüche, **dadurch gekennzeichnet, dass** die Zusammensetzung pro 100 g der Zusammensetzung 25 mg bis 200 mg, bevorzugter 40 mg bis 130 mg, besonders bevorzugt 60 mg bis 90 mg, Eicosapentaensäure enthält.

9. Zusammensetzung nach irgendeinem der vorherigen Ansprüche, **dadurch gekennzeichnet, dass** die Zusammensetzung pro 100 g der Zusammensetzung 150 mg bis 3000 mg, bevorzugter 500 mg bis 1500 mg, besonders bevorzugt 750 mg bis 1250 mg, an Isoflavonen, vorzugsweise an Soyaisoflavonen, enthält.

10. Zusammensetzung nach irgendeinem der vorherigen Ansprüche, **dadurch gekennzeichnet, dass** die Zusammensetzung pro 100 g der Zusammensetzung 0,01 g bis 1 g, bevorzugter 0,1 g bis 0,6 g, besonders bevorzugt 0,2 bis 0,4 g, eines oder mehrerer Steviolglykoside enthält.

11. Zusammensetzung nach irgendeinem der vorherigen Ansprüche, **dadurch gekennzeichnet, dass** die Zusammensetzung pro 100 g der Zusammensetzung 1 g bis 10 g, bevorzugter 1 g bis 6 g, besonders bevorzugt 2 g bis 4 g, gesättigte Fettsäuren enthält.

12. Zusammensetzung nach irgendeinem der vorherigen Ansprüche, **dadurch gekennzeichnet, dass** die Zusammensetzung pro 100 g der Zusammensetzung
50 mg bis 175 mg, besonders bevorzugt 75 mg bis 125 mg, Lutein und/oder
3 mg bis 10 mg, besonders bevorzugt 5 mg bis 7 mg, Zeaxanthin und/oder
1500 mg bis 3500 mg, besonders bevorzugt 2000 mg bis 3000 mg, Docosahexaensäure und/oder
300 mg bis 1000 mg, besonders bevorzugt 500 mg bis 700 mg, Coenzym Q10, enthält.

13. Kit, enthaltend eine erste Zusammensetzung nach irgendeinem der vorherigen Ansprüche und eine zweite pulverförmige Zusammensetzung, die ein Nahrungsergänzungsmittel und/oder Nahrungsmittel ist.

14. Verwendung der Zusammensetzung nach irgendeinem der Ansprüche 1 bis 12 oder des Kits nach Anspruch 13 als Nahrungsergänzungsmittel und/oder Nahrungsmittel, wobei von der (ersten) Zusammensetzung an einem Tag 1 g bis 10 g, bevorzugter 2 g bis 8 g, besonders bevorzugt 4 g bis 6 g, eingenommen werden.

15. Verfahren zur Herstellung einer Lösung zur oralen Applikation, **dadurch gekennzeichnet, dass** eine Zusammensetzung nach irgendeinem der Ansprüche 1 bis 12 und gegebenenfalls eine zweite pulverförmige Zusammensetzung, die ein Nahrungsergänzungsmittel und/oder Nahrungsmittel ist, in Wasser gelöst wird.

## Claims

1. Powdered composition, containing per 100 g of the composition
25 mg to 250 mg of lutein,
2 mg to 12 mg of zeaxanthin,
1000 mg to 5000 mg of docosahexaenoic acid,
100 mg to 1200 mg of coenzyme Q10 and
30 g to 90 g of one or more excipients selected from thickeners, emulsifiers and stabilizers.

2. Composition according to claim 1, **characterized in that** the one or more excipients are selected from gum arabic, alginic acid, alginates, agar-agar, carrageenan, locust bean gum, guar gum, tragacanth, xanthan gum, karaya gum, tara gum, gellan, starches, starch phosphates and gelatin.

3. Composition according to claim 1 or 2, **characterized in that** the composition contains as excipient 30 g to 90 g, preferably 40 g to 80 g, more preferably 50 g to 70 g, of gum Arabic per 100 g of the composition.

4. Composition according to any one of the preceding claims, **characterized in that** the composition is water-soluble.

5. Composition according to any one of the preceding claims, **characterized in that** the composition contains 1 g to 10 g, preferably 1 g to 6 g, more preferably 2 g to 4 g, of sugar per 100 g of the composition.

6. Composition according to any one of the preceding claims, **characterized in that** the composition contains 0.1 g to 10 g, preferably 0.3 g to 7 g, more preferably 0.4 g to 6 g, of red ginseng (Panax ginseng) extract per 100 g of the composition.

7. Composition according to any one of the preceding claims, **characterized in that** the composition contains 0.5 g to 5 g, preferably 1 g to 3 g, of acidifying agent, preferably citric acid, per 100 g of the composition.

8. Composition according to any one of the preceding claims, **characterized in that** the composition contains 25 mg to 200 mg, preferably 40 mg to 130 mg, more preferably 60 mg to 90 mg, of eicosapentaenoic acid per 100 g of the composition.

9. Composition according to any one of the preceding claims, **characterized in that** the composition contains 150 mg to 3000 mg, preferably 500 mg to 1500 mg, more preferably 750 mg to 1250 mg, of isoflavones, preferably soyaisoflavones, per 100 g of the composition.

10. Composition according to any one of the preceding claims, **characterized in that** the composition contains 0.01 g to 1 g, preferably 0.1 g to 0.6 g, more preferably 0.2 to 0.4 g, of one or more steviol glycosides per 100 g of the composition.

11. Composition according to any one of the preceding claims, **characterized in that** the composition contains 1 g to 10 g, preferably 1 g to 6 g, more preferably 2 g to 4 g, of saturated fatty acids per 100 g of the composition.

12. Composition according to any one of the preceding claims, **characterized in that** the composition contains 50 mg to 175 mg, more preferably 75 mg to 125 mg, of lutein and/or 3 mg to 10 mg, more preferably 5 mg to 7 mg, of zeaxanthin and/or 1500 mg to 3500 mg, more preferably 2000 mg to 3000 mg, of docosahexaenoic acid and/or
300 mg to 1000 mg, more preferably 500 mg to 700 mg, of coenzyme Q10 per 100 g of the composition.

13. Kit, containing a first composition according to any one of the preceding claims and a second powdered composition which is a supplement and/or food.

14. Use of the composition according to any of claims 1 to 12 or the kit according to claim 13 as a supplement and/or food product, wherein from 1 g to 10 g, preferably from 2 g to 8 g, more preferably from 4 g to 6 g, of the (first) composition is taken in one day.

15. Method for preparing a solution for oral administration, **characterized in that** a composition according to any one of claims 1 to 12 and optionally a second powder composition which is a supplement and/or food product is dissolved in water.

## Revendications

1. Composition pulvérulente, contenant, pour 100 g de la composition,
25 mg à 250 mg de lutéine,
2 mg à 12 mg de zéaxanthine,
1000 mg à 5000 mg d'acide docosahexaénoïque,
100 mg à 1200 mg de coenzyme Q10 et
30 g à 90 g d'un adjuvant ou de plusieurs adjuvants, sélectionnés parmi les agents épaississants, les émulsifiants et les stabilisateurs.

2. Composition selon la revendication 1, **caractérisée en ce que** l'un ou les plusieurs adjuvants sont sélectionnés parmi la gomme arabique, l'acide alginique, les alginates, l'agar-agar, la carraghénane, la gomme de caroube, la gomme de guar, le tragant, le xanthane, la gomme de karaya, la gomme de tara, le Gellane, les amidons, les phosphates d'amidon et la gélatine.

3. Composition selon la revendication 1 ou 2, **caractérisée en ce que** la composition contient comme adjuvant, pour 100 g de la composition, 30 g à 90 g, plus préférentiellement 40 g à 80 g, de manière particulièrement préférentielle 50 g à 70 g, de gomme arabique.

4. Composition selon l'une quelconque des revendications précédentes, **caractérisée en ce que** la composition est soluble dans l'eau.

5. Composition selon l'une quelconque des revendications précédentes, **caractérisée en ce que** la composition contient, pour 100 g de la composition, 1 g à 10 g, plus préférentiellement 1 g à 6 g, de manière particulièrement préférentielle 2 g à 4 g, de sucre.

6. Composition selon l'une quelconque des revendications précédentes, **caractérisée en ce que** la composition contient, pour 100 g de la composition, 0,1 g à 10 g, plus préférentiellement 0,3 g à 7 g, de manière particulièrement préférentielle 0,4 g à 6 g, d'extrait de ginseng rouge (ginseng de Panax).

7. Composition selon l'une quelconque des revendications précédentes, **caractérisée en ce que** la composition contient, pour 100 g de la composition, 0,5 g à 5 g, plus préférentiellement 1 g à 3 g, d'acidifiant, de préférence d'acide citrique.

8. Composition selon l'une quelconque des revendications précédentes, **caractérisée en ce que** la composition contient, pour 100 g de la composition, 25 mg à 200 mg, plus préférentiellement 40 mg à 130 mg, de manière particulièrement préférentielle 60 mg à 90 mg, d'acide eicosapentaénoïque.

9. Composition selon l'une quelconque des revendications précédentes, **caractérisée en ce que** la composition contient, pour 100 g de la composition, 150 mg à 3000 mg, plus préférentiellement 500 mg à 1500 mg, de manière particulièrement préférentielle 750 mg à 1250 mg, d'isoflavones, de préférence d'isoflavones de soja.

10. Composition selon l'une quelconque des revendications précédentes, **caractérisée en ce que** la composition contient, pour 100 g de la composition, 0,01 g à 1 g, plus préférentiellement 0,1 g à 0,6 g, de manière particulièrement préférentielle 0,2 à 0,4 g, d'un ou de plusieurs stéviolglycosides.

11. Composition selon l'une quelconque des revendications précédentes, **caractérisée en ce que** la composition contient, pour 100 g de la composition, 1 g à 10 g, plus préférentiellement 1 g à 6 g, de manière particulièrement préférentielle 2 g à 4 g, d'acides gras saturés.

12. Composition selon l'une quelconque des revendications précédentes, **caractérisée en ce que** la composition contient, pour 100 g de la composition, 50 mg à 175 mg, de manière particulièrement préférentielle 75 mg à 125 mg, de lutéine et/ou 3 mg à 10 mg, de manière particulièrement préférentielle 5 mg à 7 mg, de zéaxanthine et/ou 1500 mg à 3500 mg, de manière particulièrement préférentielle 2000 mg à 3000 mg, d'acide docosahexaénoïque et/ou
300 mg à 1000 mg, de manière particulièrement préférentielle 500 mg à 700 mg, de coenzyme Q10.

13. Kit, contenant une première composition selon l'une quelconque des revendications précédentes et une deuxième composition pulvérulente qui est un complément alimentaire et/ou un aliment.

14. Utilisation de la composition selon l'une quelconque des revendications 1 à 12 ou du kit selon la revendication 13 comme complément alimentaire et/ou aliment, sachant que, de la (première) composition, sur un jour, 1 g à 10 g, plus préférentiellement 2 g à 8 g, de manière particulièrement préférentielle 4 g à 6 g, sont ingérés.

15. Procédé de fabrication d'une solution pour administration orale, **caractérisé en ce qu'**une composition selon l'une quelconque des revendications 1 à 12 et le cas échéant une deuxième composition pulvérulente qui est un complément alimentaire et/ou un aliment et/sont dissous dans l'eau.
